# EUROPEAN PATENT APPLICATION

(11) **EP 4 556 557 A1**
(43) Date of publication of application: **21.05.2025**
(21) Application number: 23943068.9
(22) Date of filing: 05.07.2023
(51) Int. Cl.: C12N 5/0784, A61K 39/00, A61P 35/00

(54) **METHOD FOR CULTURING DENDRITIC CELLS BY FIRST EXPANDING AND THEN DIFFERENTIATING PERIPHERAL BLOOD, AND USE**

(30) Priority: 25.06.2023 CN 202310752191
(71) Applicant: Double-dove Biotechnology (Shanghai) Co. Ltd, Shanghai 201318 (CN)
(72) Inventor: WU, Hongjin, Shanghai 201318 (CN); LING, Xia, Tokyo (JP); LI, Ying, Shanghai 201318 (CN)
(74) Representative: Murgitroyd & Company
(86) International application number: PCT/CN2023/105977
(87) International publication number: WO 2025/000580

(57) **Abstract**

A method for culturing dendritic cells through amplification and differentiation of peripheral blood. After peripheral blood mononuclear cells (PBMCs) obtained by isolation are expanded *in vitro* with a cytokine composition (consisting of FLT-3L, TPO, SCF, IL3, IL6 and SR1) in an early stage and then differentiated (FLT-3L, GM-CSF, IL3 and TGF-β) to obtain cDC1s. It is verified that a method for preparing cDC1s *in vitro* of the present invention can obtain XCR1⁺ cDC1s with the number at least of 0.8×10⁶ (32%) from the starting 2.5×10⁶ PBMCs, thereby meeting the requirement of clinical application for the cell number.

## Description

### TECHNICAL FIELD

The present invention relates to a method for preparing biological materials, and in particular to a method for culturing conventional type 1 dendritic cells (cDC1s) from peripheral blood mononuclear cells (PBMCs).

### BACKGROUND

Dendritic cells (DCs), as the most powerful dedicated antigen presenting cells (APCs) in the body, play a key role in innate immune response and induced adaptive immune response. The DCs are crucial to the initiation, regulation and maintenance of the immune responses, and govern various functions of the body's immune system. Immature DCs in the body have strong migration ability, while mature DCs can effectively activate initial T cells. The DCs efficiently ingest, process and present antigens *in vivo,* and provide, through T helper (Th) cells and B cells, a recognition basis for the initiation of a specific immune response.

The DCs belong to mononuclear phagocytes (MPs) and can be divided into conventional DCs (cDCs), plasmacytoid DCs (pDCs) and Langerhans cells (LCs) according to their different sources and features of differentiation pathways. The cDCs are further divided into conventional type 1 DCs (cDC1) and conventional type 2 DCs (cDC2). The development and differentiation of the cDC1 depend on transcription factors BATF3 and IRF8. Common surface markers mainly include XCR1, CD141, CLEC9A, CADM1 and the like. The cDC1 has been confirmed to be the only antigen presenting cell that can transport an intact antigen to lymph nodes and stimulate tumor-specific CD8+ T cells. The migration and immune activation functions play a crucial role in anti-tumor immune response.

In peripheral blood, the mature DCs only account for about 0.2% of PBMCs, and the cDC1s only account for about 2.6% of the DCs. Since the content of the DCs is extremely low, the DCs cannot be obtained directly by isolation, which greatly hinders its direct use in cell therapy. Therefore, it is a main difficulty in clinical application to obtain the DCs by *in-vitro* cell differentiation and expansion. In current clinical studies, the commonly used DCs are monocyte-derived DCs (moDCs, shown as CD11b⁺) obtained by expanding and differentiating peripheral blood mononuclear cells or hemapietic stem cells (HSCs). Clinical studies have found that the moDCs has many disadvantages that they cannot effectively migrate to tumor to drain lymph node drainage and cannot directly present antigens to host T cells, but are the source of antigens, and need to be transferred and processed by cDC1s in the host responsible for directly presenting antigens to CD8⁺ T and CD4⁺ T cells, which results in poor clinical therapeutic effect.

Therefore, providing a technology that can obtain the cDC1s *in vitro* will greatly promote the research, development and use of DC cell tumor vaccines.

### SUMMARY

An objective of the present invention is to provide a method for preparing cDC1s *in vitro* to culture dendritic cells by first expanding and then differentiating peripheral blood mononuclear cells, thereby increasing the number of obtainable cDC1s and meeting the requirements of clinical application.

Another objective of the present invention is to provide a method for preparing cDC1s *in vitro* to select a combination of various cytokines and culture dendritic cells by first expanding and then differentiating peripheral blood mononuclear cells, thereby improving the efficiency for obtaining the cDC1s.

One more objective of the present invention is to provide a method for preparing cDC1s *in vitro* to more conveniently obtain the cDC1s.

Another one objective of the present invention is to provide a method for preparing cDC1s *in vitro,* use of the method in the preparation of a dendritic cell vaccine, and particularly use of the method in the preparation of a drug for *in-vitro* specific T cells.

A method for preparing cDC1s in vitro comprises co-culturing peripheral blood mononuclear cells obtained by isolation with a first cytokine composition *in vitro* to first obtain precursor cells thereof, then co-culturing the precursor cells with a second cytokine composition for differentiation so as to obtain the cDC1s, that is an HSC-2D culture method.

The first cytokine composition used in the method for preparing cDC1s *in vitro* is used in an expansion phase of the precursor cells and consists of FLT-3L, TPO, SCF, IL3, IL6 and SR1.

The second cytokine composition used in the method for preparing cDC1s *in vitro* is used in a differentiation phase of the precursor cells into the cDC1s and consists of FLT-3L, GM-CSF and TGF-β.

In an implementation of the first cytokine composition used in the method for preparing cDC1s *in vitro* of the present invention, 50±5 ng/ml of FLT-3L, 20±2 ng/ml of TPO, 20±2 ng/ml of SCF, 10±1 ng/ml of IL3, 10±1 ng/ml of IL6 and 1.0±0.1 nmol/ml of SR1 are added to a StemSpan^{™}-XF medium so as to implement the cell expansion in an early stage.

In an implementation of the second cytokine composition used in the method for preparing cDC1s *in vitro* of the present invention, cytokines of FLT-3L, GM-CSF, TGF-β and the like are added to an ImmunoCult^{™}-ACF dendritic cell medium, and the concentration of each cytokine is 50±5 ng/ml of FLT-3L, 2.5±0.2 ng/ml of GM-CSF and 10±1 ng/ml of TGF-β to perform the cell differentiation. The amount of the TGF-β is explored by the present invention and too low concentration of the TGF-β is detrimental to the differentiation suspension of the cDC1s.

In another implementation of the second cytokine composition used in the method for preparing cDC1s *in vitro* of the present invention, IL-3 is further added and the concentration thereof in the dendritic cell medium is 5.0±0.5 ng/ml.

In the method for preparing cDC1s in vitro of the present invention, plasma is further added and is from, for example, the same individuals from which the PBMCs are obtained, with the amount of, for example, 5 v/v%. No plasma, a platelet lysate (instead of plasma) and 5 v/v% autologous plasma are tested for cell culture. It is found that almost no cells survive in the serum-free state, and cells can be expanded and differentiated under the condition of the platelet lysate, but the number and the proportion of XCR1⁺ are obviously lower than those of the autologous plasma group.

In the method for preparing cDC1s *in vitro* of the present invention, for example, in 6-well plates, the inoculation amount of the PBMCs is counted as the cell density, for example, preferably 1-1.5×10⁶ cells/ml.

The method for preparing cDC1s *in vitro* of the present invention comprises co-culturing the peripheral blood mononuclear cells obtained by isolation with the cytokine composition related with HSC-2D expansion *in vitro* (for example, cultured in a cell incubator at 37°C±0.1°C and 5%CO₂) for 12 days, replacing the culture medium and adding the cytokine composition related with differentiation, and continuously culturing the cells for 9 days so as to obtain the cDC1s.

It is verified that the method for preparing cDC1s *in vitro* of the present invention can obtain cDC1s, presenting the characteristic of XCR1⁺/CD11b⁻, with the number at least of 0.8×10⁶ (32%) from the starting 2.5×10⁶ PBMCs, thereby meeting the requirement of clinical application for the cell number.

Since the XCR1⁺ cDC1s prepared by the method account for 70%-85% and the rest cells cannot start the antigen presenting function, further purification is not needed. Therefore, the method is more convenient and has higher efficiency.

After the cDC1s prepared in the present invention are subjected to maturation stimulation (GM-CSF, CD40L, R848, Poly I:C, INF-γ and antigen peptides), the cDC1s are prepared into a cell vaccine drug (preparation) for preventing and treating tumors.

After the cDC1s prepared in the present invention are subjected to maturation stimulation (such as but not limited to GM-CSF, CD40L, R848, Poly I:C, INF-γ and antigen peptides), the cDC1s can be used for activating T cell specific immunity and preparing a drug for *in-vitro* specific T cells.

Compared with the prior art, the method for preparing cDC1s of the present invention has the following advantages:
(1) the starting material is PBMCs, magnetic bead separation is not needed, and the operation is simpler and more convenient;
(2) the obtained cDC1s (XCR1⁺/CD11b⁻) have higher proportion (greater than 70%); and
(3) the number of the obtained XCR1⁺ cDC1s is more (about 0.8×10⁶ cDC1s/2.5×10⁶ PBMCs).

### BRIEF DESCRIPTION OF THE DRAWINGS

FIG. 1 shows the results of bright fields of cell morphology at various culture times under the condition of various cytokines;
FIG. 2 shows the results of expressions of cell surface markers CD34, CD141 and CD14 at an HSC-2D expansion phase under the condition of various cytokines detected by flow cytometry;
FIG. 3 shows the expansion folds of each type of cells cultured on Day 12 under the condition of various cytokines;
FIG. 4 shows the results of the expressions of the cell surface markers CD34, CD141 and XCR1 at the HSC-2D expansion phase under the conditions of various cytokines detected by flow cytometry and cell plating density;
FIG. 5 shows the results of the expressions of the cell surface markers CD34 and XCR1 at 9 days of differentiation after the HSC-2D expansion under the conditions of various cytokines detected by flow cytometry and cell plating density;
FIG. 6 is a schematic diagram showing the proportion and number of XCR1⁺ cDC1 s finally obtained under the conditions of various cytokines and cell plating density;
FIG. 7A shows the results of expressions of cell surface markers CD141 and XCR1 of cDC1s, detected by flow cytometry, randomly cultured from a female subject;
FIG. 7B shows the results of the expressions of the cell surface markers CD141 and XCR1 of cDC1s, detected by flow cytometry, randomly cultured from another male subject;
FIG. 8 shows the results of expressions of CD86 and CD83 after maturation stimulation of cDC1s detected by flow cytometry; and
FIG. 9 shows the results of expressions of specific antigen tetramers after co-culture of cDC1-T cells detected by flow cytometry.

### DETAILED DESCRIPTION

The technical solutions of the present invention are described below with reference to the accompanying drawings. The examples of the present invention are only intended to explain, rather than to limit the technical solutions of the present invention. Although the present invention is described in detail with reference to the preferred examples, a person skilled in the art should understand that modifications or equivalent substitutions may be made to the technical solutions of the present invention without departing from the spirit and scope of the technical solutions of the present invention, and such modifications or equivalent substitutions should be included within the scope of the claims of the present invention.

Various test methods used in the following examples of the present invention are specified as follows:

### 1) PBMCs obtained by isolating peripheral blood

A freshly collected blood in a heparin sodium anticoagulation tube is subjected to PBMC isolation, the blood collection tube is slightly inverted for several times, and the evenly mixed whole blood is added into a centrifuge tube containing a Ficoll lymphocyte separation solution according to the ratio of 1:1 and centrifuged at 2,500 rpm for 15 minutes. After the centrifugation, an upper yellow plasma fraction is transferred to a new centrifuge tube and inactivated at 56°C for 30 minutes before use. A buffy coat is gently pipetted into a 15-ml centrifuge tube, a PBS buffer is added, and the materials are evenly mixed and centrifuged at 2,500 rpm for 10 minutes. After the centrifugation, liquid is discarded, a cell precipitate at the bottom of the tube is blown up by using an erythrocyte lysate, and the erythrocyte is lysed for 10 minutes. After the completion of the lysis, the lysed cells are centrifuged at 2,500 rpm for 5 minutes. At this time, the cell precipitate is resuspended in a buffer PBS and counted. Finally, the suspension is centrifuged at 2,500 rpm for 5 minutes and the supernatant was discarded to obtain PBMCs.

### 2) Flow cytometry detection method

In order to identify the cell components during the culture process and the proportion of cDC1s in the suspended cells that are finally expanded and differentiated, the surface markers are detected by flow cytometry in the present example. The used antibodies are Human XCR1 PE-conjugated antibody (R&D Systems, FAB8571P-100), APC mouse anti-human CD34 (BD, 555824), PE mouse anti-human CD141 (BD, 559781), APC mouse anti-human CD83 (BD, 551073), PerCP-Cy5.5 mouse anti-human CD86 (BD, 561129), Tritest CD3/4/8 (BD, 340298) and HLA-A*02:01 GPC3 Tetramer-FVGEFFTDV-APC (MBL, TB-0134-2).

### 3) Method for counting total number of cells

In the example, the calculation method of the number of cells in each group is as follows: all the suspended cells in 6-well plates are counted 12 days after HSC-2D expansion or all the suspended cells in 6-well plates are counted after HSC-2D expansion for 12 days and then differentiation for 9 days.

### Example 1 Selection of cytokines of cDC1s obtained from PBMCs by first HSC-2D expansion and then differentiation

On the basis of an experiment explored at an early stage of cell culture of cDC1s, several cytokines were preliminarily determined. To further explore the conditions for the cell culture of the cDC1s, the present example first tested in four groups of different concentrations of the cytokines. The PBMCs obtained from the isolation were plated evenly into the following groups of culture conditions:
(1) FST36-H group: FLT-3L (100 ng/ml), SCF (100 ng/ml), TPO (50 ng/ml), IL3 (20 ng/ml) and IL6 (20 ng/ml);
(2) FST36-L group: FLT-3L (50 ng/ml), SCF (20 ng/ml), TPO (20 ng/ml), IL3 (10 ng/ml) and IL6 (10 ng/ml);
(3) FST36-H+SR1 group: FLT-3L (100 ng/ml), SCF (100 ng/ml), TPO (50 ng/ml), IL3 (20 ng/ml), IL6 (20 ng/ml) and SR1 (1 nmol/ml); and
(4) FST36-H+SR1+VEGF group: FLT-3L (100 ng/ml), SCF (100 ng/ml), TPO (50 ng/ml), IL3 (20 ng/ml), IL6 (20 ng/ml), SR1 (1 nmol/ml) and VEGF (2 ng/ml).

Since DCs were mainly derived from the differentiation of HSCs and precursor cells thereof, in order to obtain sufficient numbers of DC precursor cells, the initial culture was an HSC-2D expansion phase. The culture medium used in this phase was StemSpan^{™}-XF (STEMCELL, #100-0073). During use, the culture medium contained 5 v/v% of autologous plasma and 1 w/v% of penicillin-streptomycin. The cell plating density was 1×10⁶/ml and each condition group was plated in two duplicate wells of 6-well plates with 2 ml of the culture medium per well, i.e., the number of cell plating per well was 2×10⁶. After the culture was finished, the plates were placed in a cell incubator at 37°C±0.1°C and 5%CO₂. After the cells were cultured for 2 hours, the culture medium was changed once. Then the culture medium was changed or supplemented every 2-3 days according to the cell growth state, and the cells were regularly observed and photographed.

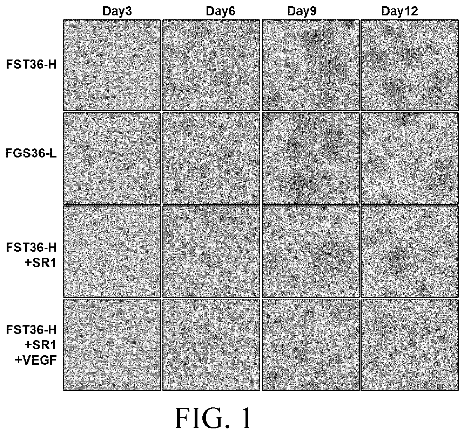

The time of day of the cell plating was recorded as Day 0. As the culture time was prolonged, the cells were gradually increased and aggregated from the early adhesion state. The cell block mass gradually formed at the beginning of the culture on Day 6. At this time, the suspended cells were mostly in the form of small cells. The small cells gradually became fewer as the culture progressed. The cell block mass was obvious on Day 9. Then the block mass would be suspended and dispersed into individual cells, as shown in FIG. 1. It can be seen from FIG. 1 that the cell growth was the slowest in the VEGF-added group, while there was no significant difference among the other three groups.

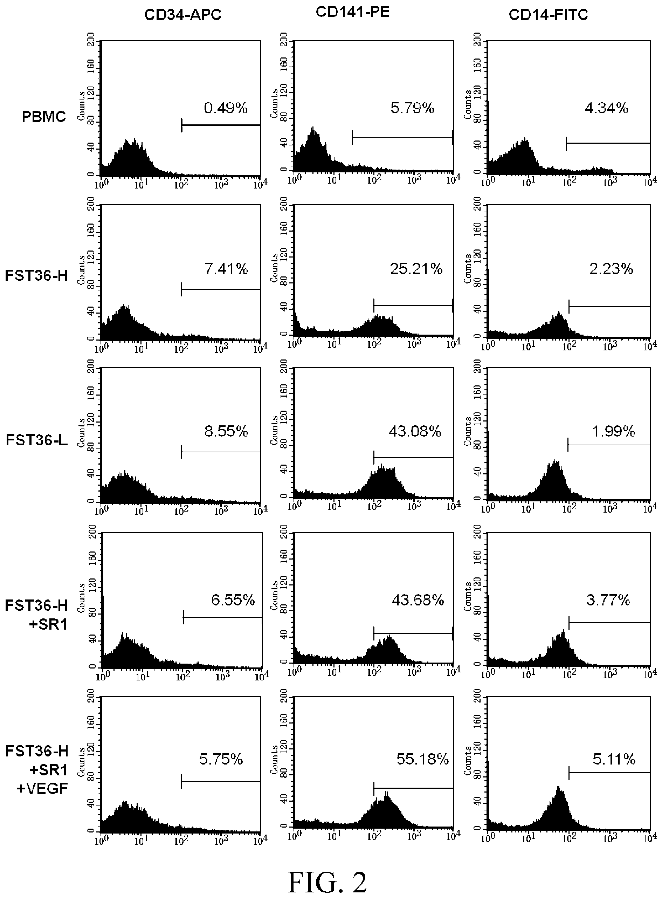

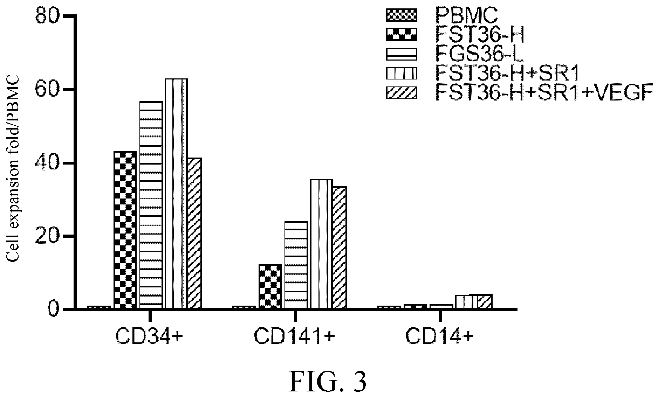

After 12 days of the expansion culture, the suspended cells in the culture wells were taken for flow cytometry of CD34 (HSC surface marker), CD141 (DC surface marker) and CD14 (monocyte surface marker). Comparing the FTS36-H group with the FTS36-L group, it can be seen that the expression of CD141 was significantly higher in the FTS36-L group (43.08%) than that in the FTS36-H group (25.21%); and comparing the FTS36-H group with the FTS36-H+SR1 group, the expression of CD141 was higher in the FTS36-H+SR1 group (43.68%) than that in the FTS36-H group (25.21%) (see FIG. 2). Although the expression of CD141 was also high in the FTS36-H+SR1+VEGF group, its CD34 expression was slightly lower than that of the other groups and its cell growth was slow. Therefore, the group did not have significant advantages. The expression of CD34 was significantly increased in each group compared to PBMC with the expansion fold of 40-fold or more (see FIG. 3), indicating that the cytokine composition was effective in expanding HSCs. Besides, the expansion fold of the FTS36-L group and the FTS36-H+SR1 group was higher than that of the other two groups. There was little expansion of CD14, indicating that the culture conditions were not favorable for monocyte proliferation.

In conclusion, the appropriate cytokine condition was determined to be FTS36-L+SR1.

### Example 2 Selection of cell plating density of cDC1s obtained from PBMCs by first HSC-2D expansion and then differentiation

The PBMCs obtained from the isolation were plated according to different densities into the following groups of culture conditions:
(1) FST36S-H group: FLT-3L (100 ng/ml), SCF (100 ng/ml), TPO (50 ng/ml), IL3 (20 ng/ml), IL6 (20 ng/ml) and SR1 (1 nmol/ml); and
(2) FST36S-L group: FLT-3L (50 ng/ml), SCF (20 ng/ml), TPO (20 ng/ml), IL3 (10 ng/ml), IL6 (10 ng/ml) and SR1 (1 nmol/ml).

The plating density was 0.5×10⁶/ml, 1.25×10⁶/ml and 2.5×10⁶/ml respectively. Each condition group was plated in two duplicate wells of 6-well plates with 2 ml of the culture medium per well The initial culture was an HSC-2D expansion phase. The culture medium used in this phase was StemSpan^{™}-XF(STEMCELL, #100-0073). After the expansion for 12 days, the culture medium was replaced into an ImunoCult^{™}-ACF dendritic cell medium (STEMCELL, #10986) for continuous differentiation culture for 9 days. The cytokines used in the differentiation were as follows: FLT-3L (50 ng/ml), GM-CSF (2.5 ng/ml), IL3 (5 ng/ml) and TGF-β (10 ng/ml). During use, the both culture mediums contained 5% of autologous plasma and 1 w/v% of penicillin-streptomycin. After the plating was finished, the plates were placed in a cell incubator at 37°C±0.1°C and 5%CO₂. After the cells were cultured for 2 hours, the culture medium was changed once. Then the culture medium was changed or supplemented every 2-3 days according to the cell growth state, and the cells were regularly observed and photographed.

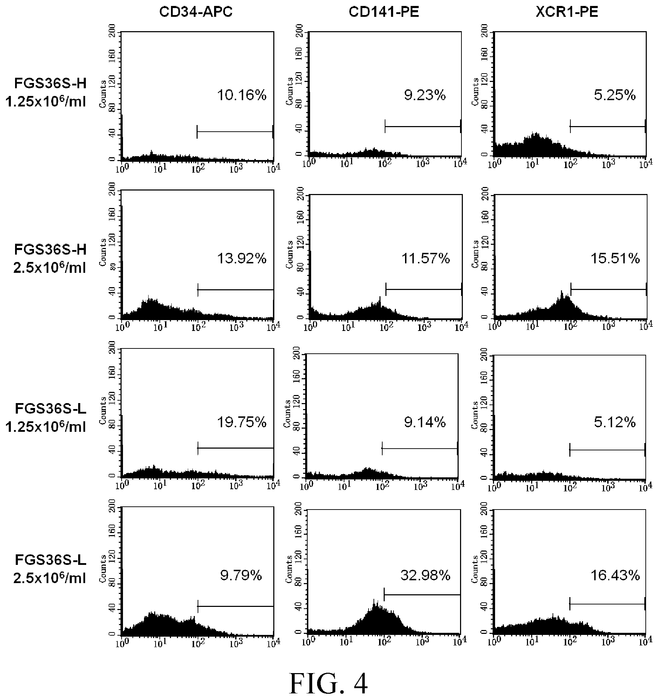

After 12 days of the expansion culture, the suspended cells in the culture wells were taken for flow cytometry of CD34, CD141 and XCR1. Since CD141 was expressed on various types of DCs, while XCR1 was only expressed on cDC1s, XCR1 was subsequently selected to characterize the cDC1s. At this time, in the 0.5×10⁶/ml plating density group, since the cells grew relatively slow, there was no cell suspension. Therefore, flow cytometry cannot be performed. Comparing the two groups with the density of 1.25×10⁶/ml, the CD34 expression proportion was higher in the FST36S-L group (19.75%) than the FST36S-H group (10.16%). There was no difference in the XCR1 proportion between the two groups. Comparing the two groups with the density of 2.5×10⁶/ml, the CD141 expression proportion was higher in the FST36S-L group (32.98%) than the FST36S-H group (11.57%). There was almost no difference in the XCR1 proportion between the two groups (see FIG. 4).

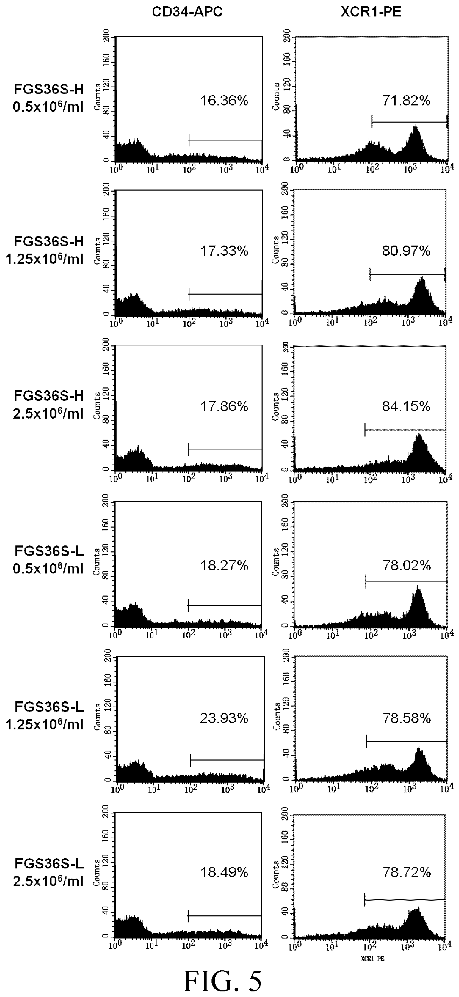

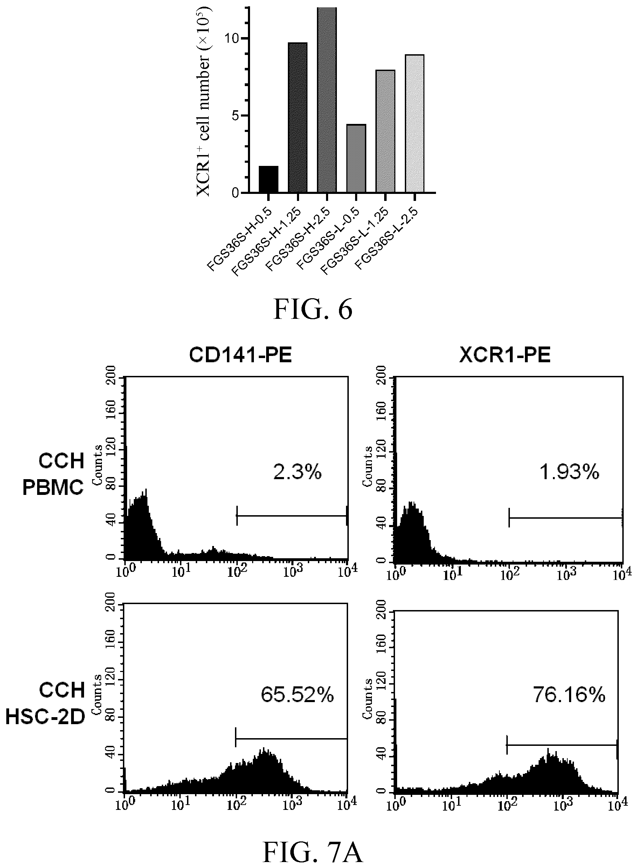

After 12 days of the expansion culture, the culture medium was replaced with a differentiation culture medium. After differentiation culture was continuously performed for 9 days, the cells were harvested. The suspended cells in the culture wells were taken for flow cytometry of CD34 and XCR1. The expression of CD34 was not very different among the groups. The expression of XCR1 was about 80% of other groups except the 0.5×10⁶/ml FST36S-H group (71.82%) (see FIG. 5). The number of finally obtained XCR1⁺ cDC1s was analyzed and compared (see FIG. 6). It can see that comparing the plating density of 2.5×10⁶/ml with that of 1.25×10⁶/ml, the obtained cell number was only slightly higher, not multiplied. Comparing the plating density of 0.5×10⁶/ml with that of 1.25×10⁶/ml, the cell number in the FST36S-H group was too low at the low density. Comparing the plating density of 0.5×10⁶/ml and that of 1.25×10⁶/ml in the FST36S-L group, the ratio of the number of the finally harvested cells was approximately 2 times. Therefore, it was estimated that the plating density of 1.25×10⁶/ml was the optimum density. In addition, although the number of the cells in the FST36S-H group was slightly greater than that in the FST36S-L group, the difference was small. Besides, comprehensively considering the cost, FST36S-L was the optimum condition for the expansion culture of XCR1⁺ cDC1s.

In condition, it was determined suitable conditions for HSC-2D expansion and differentiation of PBMCs were:
(1) in the HSC-2D expansion phase, FST36S-L: FLT-3L (50 ng/ml), SCF (20 ng/ml), TPO (20 ng/ml), IL3 (10 ng/ml), IL6 (10 ng/ml) and SR1 (1 nmol/ml), and a StemBan^{™}-XF culture medium were used; and
(2) in the differentiation phase, FG3T: FLT-3L (50 ng/ml), GM-CSF (2.5 ng/ml), IL3 (5 ng/ml) and TGF-β (10 ng/ml), and an ImunoCult^{™}-ACF dendritic cell medium were used. The cell plating density was preferably about 1.25×10⁶/ml.

### Example 3 Stability of method for obtaining cDC1s from PBMCs by HSC-2D expansion and then differentiation

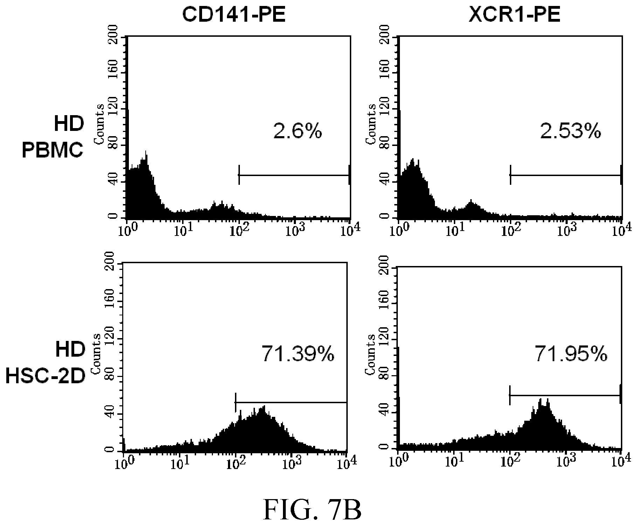

To verify the repeatability of the above culture method, two volunteers (one male and one female) were randomly selected and subjected to a XCR1⁺ cDC1 culture test. Differentiation was performed for 12 days using FST36S-L followed by differentiation for 9 days using FG3T. The whole process of the co-culture was 21 days. The cell plating density was about 1.25×10⁶/ml. The results of flow cytometry of the finally harvested cells were shown in FIGs. 7A and 7B. FIG. 7A was a female subject with the proportion of XCR1⁺ of 76.16%; and FIG. 7B was a male subject with the proportion of XCR1⁺ of 71.95%. The results indicated that the method was stable and repeatable and had universal significance.

### Example 4 Antigen-presenting function of cDC1s obtained from PBMCs by HSC-2D expansion and differentiation

Method: To verify whether XCR1⁺ cDC1s obtained by *in-vitro* culture had an antigen-presenting function, cDC1s were subjected to maturation stimulation and an antigen loading test. The resuscitated cDC1s were plated under the following conditions: 20 ng/ml of GM-CSF, 1 µg/ml of CD40L, 10 µg/ml of R848, 20 µg/ml of Poly I:C, 10 ng/ml of INF-γ, 10 ng/ml of IL-1β and 5 ng/ml of TNF-α (the cytokines of IL-1β and TNF-α can be removed by an experimental test, which had almost no influence on the maturation stimulation of DCs), and still an ImunoCult^{™}-ACF dendritic cell medium. The cells were cultured overnight (16-24 hours, no more than 48 hours) in a cell incubator at 37°C±0.1°C and 5%CO₂. On the second day, 20 µg/ml of a GPC3 antigenic peptide (FVGEFFTDV) was added and the cells were continuously cultured for 2 hours. If the cDC1s, resuscitated after cryopreservation, plated in a common culture dish, the cDC1s would become an adherent state. Therefore, the cells were plated in a low adsorption culture dish or directly in an original culture dish after HSC-2D expansion and differentiation culture for maturation stimulation without a cryopreservation operation.

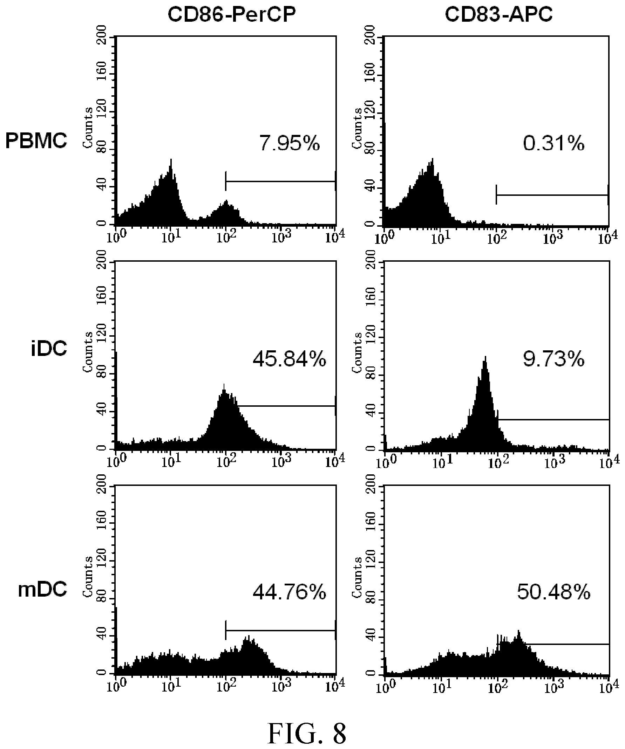

The cDC1s after the maturation stimulation and antigen loading should express surface markers such as CD83, CD86 and the like. CD83 was a sign of DC maturation and CD86 was used as a co-stimulation factor to prove that the DCs had the function of activating T cells. The expressions of CD83 and CD86 on the surfaces of the cDC1s were detected by flow cytometry. It was found that after the maturation stimulation on the cultured cDC1s, CD83 was increased from 9.73% in immature dendritic cells (iDCs) to 50.48% in mature dendritic cells (mDCs). However, the expression of CD83 in the PBMCs was only 0.31%, meaning that the cDC1s had become mature at this time. The expression of the co-stimulation factor CD86 reached 40% or more in both the iDC state and the mDC state, which was greatly improved compared with 7.95% in the PBMCs, indicating the DCs had the function of activating T cells (see FIG. 8).

T cells were cultured as follows: the PBMCs were plated on a flask coated with OKM25 (FUKUKO, Japan) one day before the co-culture, and an appropriate amount of an OKM100 medium (FUKUKO, Japan), 5% of autologous plasma and 1 /v% of penicillin-streptomycin were added. Co-culture of DC-T was divided into two groups: (1) T cell control group, i.e. the control of the same number of T cells alone without DCs; and (2) DC-T co-culture group: mature and antigen-loaded cDC1s were mixed with T cells from the same subject at a ratio of DC:T = 1:5 and plated, and the ratio may range from 1:5 to 1:20, typically suggested as 1:10. After the plating was finished, the plates were placed in a cell incubator at 37°C±0.1°C and 5%CO₂ for three days and the cells were subjected to flow cytometry.

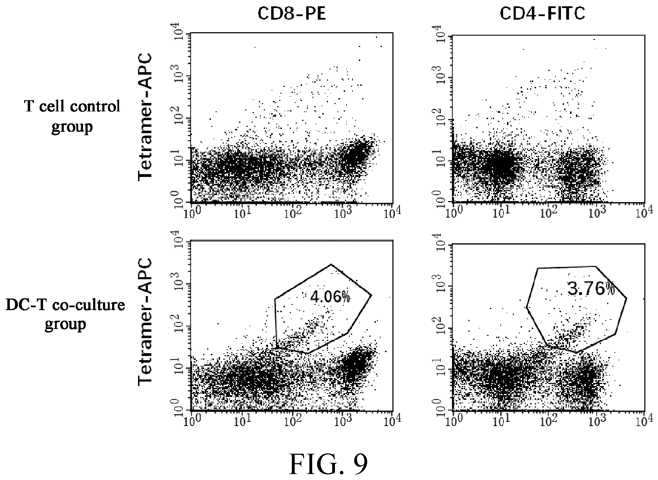

If antigen-specific T cells were generated, they would express the corresponding antigen tetramers. Therefore, the expression of GPC3 tetramers in CD4⁺ T and CD8⁺ T cells was analyzed by flow cytometry (see FIG. 9). Compared with the T cells in the control group, a small population of GPC3 tetramer positive cells were generated in both CD4⁺ T and CD8⁺ T cells, the proportion of CD4⁺/GPC3 tetramer⁺ was 3.76%, and the proportion of CD8⁺/GPC3 tetramer⁺ was 4.06%. The results indicated that the cDC1s cultured by the method can be subjected to antigen presentation and activated to generate T cells with antigen specificity.

## Claims

1. A method for preparing cDC1s *in vitro,* wherein peripheral blood mononuclear cells obtained by isolation are co-cultured with a cytokine composition *in vitro* to obtain the cDC1s;
the co-culture comprises:
co-culturing *in vitro* the peripheral blood mononuclear cells obtained by isolation with a first cytokine composition to first obtain precursor cells thereof, and
co-culturing the precursor cells with a second cytokine composition, and differentiating the cells to obtain the cDC1s;
the first cytokine composition consists of FLT-3L, TPO, SCF, IL3, IL6 and SR1; and
the second cytokine composition consists of FLT-3L, GM-CSF, IL3 and TGF-β.

2. The method for preparing cDC1s *in vitro* according to claim 1, wherein the first cytokine composition is added to a StemSpan^{™}-XF medium.

3. The method for preparing cDC1s *in vitro* according to claim 1, wherein the second cytokine composition is added to an ImmunoCult^{™}-ACF dendritic cell medium.

4. The method for preparing cDC1s *in vitro* according to claim 1, wherein the concentration of each cytokine of the first cytokine composition is 50±5 ng/ml of FLT-3L, 20±2 ng/ml of TPO, 20±2 ng/ml of SCF, 10±1 ng/ml of IL3, 10±1 ng/ml of IL6 and 1.0±0.1 nmol/ml of SR1 respectively.

5. The method for preparing cDC1s *in vitro* according to claim 1, wherein the concentration of each cytokine of the second cytokine composition is 50±5 ng/ml of FLT-3L, 2.5±0.2 ng/ml of GM-CSF, 5.0±0.5 ng/ml of IL-3 and 10±1 ng/ml of TGF-β.

6. The method for preparing cDC1s *in vitro* according to claim 1, wherein plasma is further added.

7. The method for preparing cDC1s *in vitro* according to claim 6, wherein the amount of the plasma is 5 v/v%.

8. The method for preparing cDC1s *in vitro* according to claim 1, wherein the inoculation density of the peripheral blood mononuclear cells is 1-1.5×10⁶ cells/ml.

9. The method for preparing cDC1s *in vitro* according to claim 1, wherein the peripheral blood mononuclear cells obtained by isolation are co-cultured with the cytokine composition *in vitro,* namely, first subjected to cell expansion culture for 12 days and then differentiation culture for 9 days to obtain the cDC1s.

10. Use of the method for preparing cDC1s *in vitro* according to claim 1 in the preparation of a dendritic cell vaccine.

11. Use of the method for preparing cDC1s *in vitro* according to claim 1 in the preparation of a drug for *in-vitro* specific T cells.
